# FASCICULE DE BREVET EUROPEEN

(11) **EP 1 192 282 B1**
(45) Date de publication et mention de la délivrance du brevet: **03.03.2004**
(21) Numéro de dépôt: 00949567.2
(22) Date de dépôt: 28.06.2000
(51) Int. Cl.: C12Q 1/68

(54) **PROCEDE D'ANALYSE D'UN ANIMAL PHOTOPERIODIQUE POUR UNE FONCTION PHYSIOLOGIQUE SAISONNIERE AU MOYEN DE GENOTYPAGE**
VERFAHREN ZUR ANALYSE EINER JAHRESZEITLICHEN PHYSIOLOGISCHEN FUNKTION IN EINEM FÜR LICHTSCHWANKUNGEN EMPFINDLICHEN TIER MITTELS GENTYPSIERUNG
METHOD FOR TYPING A PHOTOPERIODIC ANIMAL FOR A SEASONAL PHYSIOLOGICAL FUNCTION SUCH AS ITS CAPACITY FOR REPRODUCTION OUT OF SEASON

(30) Priorité: 28.06.1999 FR 9908238
(43) Date de publication de la demande: 03.04.2002
(73) Titulaire: INSTITUT NATIONAL DE LA RECHERCHE AGRONOMIQUE (INRA), 75338 Paris Cédex 07 (FR); CENTRE NATIONAL DE LA RECHERCHE SCIENTIFIQUE (CNRS), 75794 Paris Cedex 16 (FR); ECOLE NATIONALE SUPERIEURE D'AGRONOMIE DE MONTPELLIER E.N.S.A.M., 34060 Montpellier (FR)
(72) Inventeur: BODIN, Loys, F-31570 Tarabel (FR); PELLETIER, Jean, F-37380 Monnaie (FR); CHEMINEAU, Philippe, F-37210 Vouvray (FR); TEYSSIER, Jacques, F-34090 Montpellier (FR); HANOCQ, Eric, F-80080 Amiens (FR); THIMONIER, Jacques, F-34130 Mauguio (FR); MALPAUX, Benoît, F-37360 Beaumont-la-Ronce (FR)
(74) Mandataire: Breese, Pierre
(86) Numéro de dépôt international: PCT/FR2000/001811
(87) Numéro de publication internationale: WO 2001/000867

(56) Documents cités:
- WO-A-97/04094
- WO-A-98/03549
- BARRETT P ET AL: "Cloning and functional analysis of a polymorphic variant of the ovine Mel 1a melatonin receptor" BIOCHIMICA ET BIOPHYSICA ACTA, vol. 1356, 27 mai 1997 (1997-05-27), pages 294-307, XP000901073
- MESSER L. ET AL: "Mapping of the melatonin receptor 1a (MTNRIA) gene in pigs, sheep and cattle" MAMMALIAN GENOME, vol. 8, no. 5, - 1997 pages 368-70, XP000971344
- PELLETIER J ET AL: "Association between expression of reproductive seasonality and alleles of the gene Mel1a receptor in the ewe" BIOLOGY OF REPRODUCTION, vol. 62, avril 2000 (2000-04), pages 1096-1101, XP000971302
- EBISAWA T ET AL: "Allelic variants of human melatonin 1a receptor: function and prevalance in subjects with circadian rhythm sleep disorders" BIOCHEMICAL AND BIOPHYSICAL RESEARCH COMMUNICATIONS, vol. 262, 7 septembre 1999 (1999-09-07), pages 832-837, XP002134684
- REPPERT S ET AL: "Cloning and characterization of a mammalian melatonin receptor that mediates reproductive and circadian responses" NEURON, vol. 13, novembre 1994 (1994-11), pages 1177-85, XP000572113
- JOCKERS R ET AL: "Structure et fonction des récepteurs de la mélatonine" COMPTES RENDUS DES SEANCES DE LA SOCIETE DE BIOLOGIE ET DE SES FILIALES, vol. 192, no. 4, 13 mai 1998 (1998-05-13), pages 659-667, XP000901194

## Description

L'invention est relative au génotypage du récepteur à la mélatonine chez des animaux photopériodiques. On entend par animal photopériodique, un animal dont une fonction physiologique est assujettie aux variations annuelles de la durée du jour. En effet, le récepteur à la mélatonine est un passage obligé entre le régime lumineux et plusieurs fonctions physiologiques saisonnières, comme la reproduction par exemple chez les ovins ou les caprins ou la pousse du poil chez ces mêmes espèces. Ainsi, l'invention concerne plus particulièrement une méthode facilitant la reproduction des animaux domestiques durant l'année entière. En particulier, l'invention se rapporte à une méthode de typage d'animaux photopériodiques permettant un choix précoce des reproducteurs males ou femelles, basée sur l'identification d'un allèle nouveau du récepteur Mel₁ₐ de la mélatonine.

La reproduction de plusieurs espèces domestiques est saisonnée, ce qui signifie que les périodes d'activité sexuelle alternent avec des périodes d'inactivité apparaissant aux mêmes dates chaque année. Ces rythmes naturels de reproduction sont un frein à l'élevage car ils conduisent à des variations saisonnières de la production de lait ou d'agneaux avec des modifications correspondantes des prix. Il a donc été proposé plusieurs méthodes pour induire une reproduction en contre-saison (Malpaux et al., 1992, in "Melatonin biosynthesis, physiological effects and applications", Yu and Reiter Eds., 253-287, CRC Press, Boca Raton, USA ; Chemineau et Malpaux, 1998, Thérapie, 53, 445-452). Chez les mâles reproducteurs, des traitements photopériodiques appropriés sont susceptibles de déclencher une activité sexuelle à volonté mais impliquent un investissement lourd en bâtiments fermés. Chez la brebis, plusieurs solutions sont utilisées pour le déclenchement d'une courte saison sexuelle où les femelles peuvent être fécondées, mais chacune de ces méthodes présentent des avantages et des inconvénients :
- les traitements impliquant l'usage de progestagènes et de gonadotropine sérique (PMSG) sont efficaces, mais les craintes vis-à-vis de l'emploi de produits de synthèse limitent leur utilisation,
- les traitements combinant l'usage d'un traitement photopériodique simple, en bergerie ouverte, avec l'usage d'implant de mélatonine sont également efficaces, mais sont délaissés du fait des réserves de certains groupements d'éleveur vis-à-vis de l'emploi d'une hormone même naturelle,
- les pratiques d'élevage sans traitements hormonaux basées sur une suralimentation et l'entrée de mâles, jusque là séparés, dans les groupes de femelles sont efficaces, mais leur efficacité est variable selon les races.

Outre ces différentes méthodes de mise en oeuvre rapide, il pourrait être envisagé une méthode de sélection avec indexation des mâles reproducteurs, mais cette méthode est très difficile voire impossible à réaliser dans les élevages en ce qui concerne le saisonnement. En effet, la mesure directe de l'activité sexuelle spontanée à contre-saison est difficile car :
- elle ne s'exprime que si la femelle a mis bas depuis plus de 3 mois,
- elle est modulée par les conditions locales telle que l'alimentation,
- elle nécessite d'être mesurée plusieurs années de suite chez les mêmes femelles,
- elle requiert une logistique assez lourde de dosage de progestérone.

Les travaux de recherche effectués dans ce domaine ont donc porté depuis une quarantaine d'années, soit sur des traitements d'induction de l'activité sexuelle à contre-saison, soit sur des schémas de sélection permettant l'amélioration des races à la demande des professionnels de l'élevage. En outre, les physiologistes ont continuellement étudié d'un point de vue fondamental, le mode d'action du régime lumineux qui est le responsable majeur de la synchronisation de l'activité sexuelle. Ces recherches ont conduit à identifier la mélatonine libérée par la glande pinéale, comme un facteur majeur faisant le relais entre l'information photopériodique circadienne et annuelle et le système à GnRH hypothalamique qui est le système de commande unique de la reproduction. Deux voies ont été explorées :
- L'incidence des taux de mélatonine. Cette hormone est sécrétée la nuit mais présente des niveaux très variables selon les individus, de 20 à 1000 pg/ml en moyenne au milieu de la phase nocturne. Ce caractère a été montré très héritable (Zarazaga et al., 1998, Am. J. Physiol 274, E607-E610) mais il n'a pu être encore mis en évidence un lien avec la reproduction elle-même. Pour l'instant, il est admis que la mélatonine intervient par son mode de sécrétion nocturne, ajustée sur la durée de la nuit et renseignant ainsi l'animal sur le moment de la reproduction. La mélatonine est donc impliquée dans le saisonnement de la reproduction.
- L'incidence des récepteurs à la mélatonine récemment identifiés chez les mammifères (Reppert et al., 1994, Neuron, 13, 1177-1185). Ces récepteurs constituent le maillon immédiatement en aval de la mélatonine et, chez les mammifères, trois récepteurs ont été identifiés :
   . Mel₁ₐ (Reppert et al., 1994, Neuron, 13, 1177-1185),
   . Mel_{1aβ} (Barrett et al., 1997, Biochim. Biophys. Acta, 1356, 299-307),
   . Mel_{1b} (Reppert et al., 1995, Proc. Natl. Acad. Sci. USA, 92, 8734-8738).

Le récepteur Mel_{1b} est disqualifié pour être impliqué dans les phénomènes de saisonnement puisque l'inactivation naturelle du gène de ce récepteur chez le hamster doré et le hamster sibérien ne modifie pas le saisonnement de la reproduction chez ces espèces (Weaver et al., 1996, Mol. Endo., 10, 1478-1487).

Le récepteur Mel₁ₐ, et ses variants potentiels, est trouvé dans l'ensemble du corps et bien représenté dans le cerveau et l'hypothalamus où la mélatonine agit spécifiquement pour contrôler la reproduction (Malpaux et al., 1998, Endocrinology, 139, 1508-1516). Le récepteur Mel₁ₐ constitue actuellement le candidat le plus sérieux pour transmettre l'effet de la mélatonine vers le système GnRH.

La séquence nucléotidique correspondant au récepteur Mel₁ₐ est constitué de 2 exons séparés d'un intron volumineux (>10 kb) et la séquence codante comprend 1101 nucléotides soit 367 acides aminés dont respectivement 78 et 289 acides aminés pour les exons I et II. Il a été montré récemment l'existence de variants de la séquence de l'exon II du récepteur Mel₁ₐ chez le mouton :
- Huit mutations ponctuelles de la séquence de Reppert et al. (1994, Neuron, 13, 1177-1185) prise comme référence, ont été décrites par Barrett et al. (Barrett et al., 1997, Biochim. Biophys. Acta, 1356, 299-307) qui ont assimilé ce variant à Mel_{1aβ}. Cependant, aucune variation dans la liaison au récepteur ou de la sécrétion de l'AMP cyclique n'est observée après l'expression *in vitro* de ce récepteur dans des cellules L de souris,
- Deux mutations en position 606 et 612, ont été présentées comme entraînant respectivement la perte de sites de coupure enzymatiques RsaI et MnlI (Messer et al.,Mammalian Genome, 1997, 8, 368-370) chez différentes races d'ovins et de bovins.

L'importance de ces modifications de séquences sur la physiologie de la reproduction n'était pas connue jusqu'à la réalisation des travaux ayant conduit à la présente invention. Cependant, la modification par mutagenèse dirigée, de la séquence en acides aminés dans le 5^{ème} passage transmembranaire du récepteur Mel₁ₐ diminue l'affinité de la liaison de la mélatonine (Conway et al., 1997, Biochem., Biophys., Res. Comm., 239, 418-423).

Les inventeurs ont maintenant mis en évidence un lien entre la structure du gène du récepteur Mel₁ₐ et une fonction physiologique en relation avec le rôle joué normalement par la mélatonine notamment dans le saisonnement de la reproduction. Plus particulièrement, les travaux de recherche réalisés dans le cadre de la présente invention ont montré une relation hautement spécifique entre un génotype défini par une fraction de la séquence nucléotidique de l'exon II du récepteur Mel₁ₐ de la mélatonine, et l'expression de l'anoestrus saisonnier de la reproduction.

La présente invention a donc pour objet une méthode de typage d'un animal photopériodique pour une fonction physiologique saisonnière et/ou sa capacité à transmettre cette fonction physiologique saisonnière à sa descendance, caractérisée en ce que l'on identifie le génotype d'un site de restriction MnlI polymorphe présent dans l'Exon II du récepteur Mel₁ₐ à la mélatonine, une mutation au niveau du site de reconnaissance de ladite enzyme permettant de sélectionner les animaux susceptibles de présenter une fonction physiologique saisonnière et/ou de transmettre cette aptitude à sa descendance. L'identification dudit génotype peut être réalisée par toute technique de biologie moléculaire connue de l'homme, comme notamment l'analyse des produits de digestion par ladite enzyme.

Tout particulièrement, l'invention concerne le typage d'un animal photopériodique pour son aptitude à la reproduction à contre-saison et/ou sa capacité à transmettre cette aptitude à sa descendance, caractérisée en ce que l'on identifie le génotype d'un site de restriction MnlI polymorphe présent dans l'Exon II du récepteur Mel₁ₐ à la mélatonine, une mutation au niveau du site de reconnaissance de ladite enzyme permettant de sélectionner les animaux susceptibles de présenter une aptitude à la reproduction à contre-saison et/ou à transmettre cette aptitude à sa descendance.

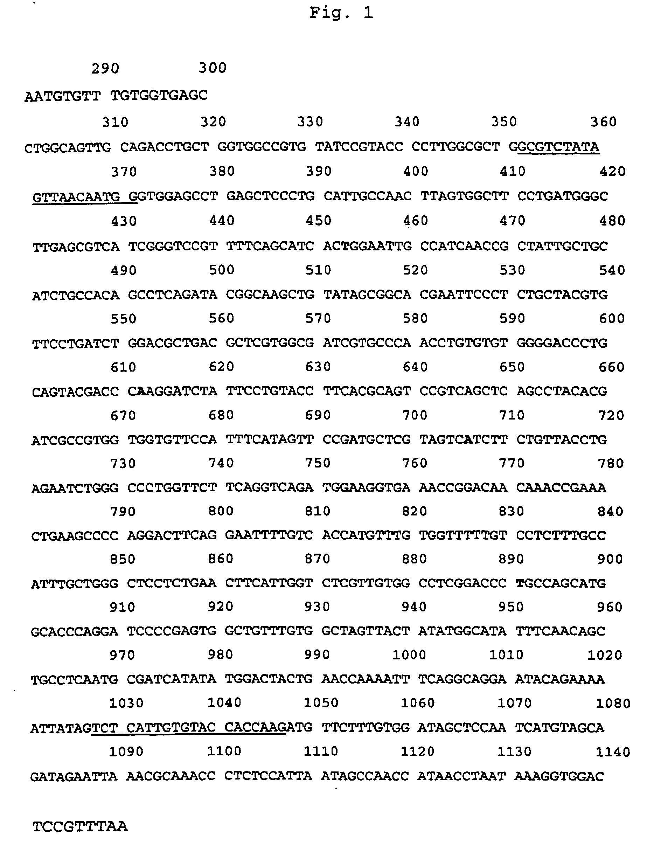

En effet, de façon surprenante, les inventeurs ont maintenant mis en évidence que la présence d'une mutation particulière, définissant un allèle "-", sur les deux chromosomes parentaux était associée avec le saisonnement de la reproduction. La séquence de l'Exon II du récepteur Mel₁ₐ de la mélatonine chez les ovins constituant la séquence de référence est celle donnée par Reppert et al. (1994, Neuron, 13, 1177-1185) représentée dans la figure 1 en annexe. Dans la figure 1, il est indiqué en gras, les mutations caractéristique de l'allèle "-" situées au niveau des nucléotides en positions 612, 453, 706 et 891, la mutation en 612 entraînant la perte du site de coupure MnlI. Les séquences soulignées dans la figure 1 correspondent aux séquences des amorces utilisées dans les protocoles de PCR décrits dans la partie expérimentale de l'invention.

Les travaux de génotypage de l'Exon II du récepteur Mel₁ₐ par l'enzyme de restriction MnlI, réalisés dans le cadre de l'invention à partir d'échantillons sanguins prélevés sur des animaux susceptibles de présenter ou non une activité sexuelle spontanée en contre-saison, ont montré, que le site de reconnaissance au niveaux des nucléotides en position 612-615 dans la figure 1 peut être affecté d'une mutation du nucléotide en position 612, consistant en le remplacement d'un nucléotide G par un nucléotide A, entraînant la perte du site de coupure au niveau du nucléotide en position 605.

Le séquençage de l'Exon II a montré que cette mutation est associée à un jeu de trois autres mutations au niveau des nucléotides en position 453, 706 et 891 dans la figure 1 définissant un allèle désigné "-", par rapport à un jeu d'allèles où le site MnlI est présent, allèles "+".

Cependant, du fait de la liaison entre ces mutations, l'absence du site de reconnaissance MnlI est suffisante pour établir la présence de l'allèle "-".

Les résultats obtenus montrent que la distribution des fréquences des génotypes "--", "+-" et "++" est différente selon que les animaux présentent ou non une activité sexuelle spontanée en contre-saison. En particulier, le génotype "--" n'a été trouvé que chez des animaux sexuellement inactifs en contre-saison mais non chez ceux qui manifestaient une activité sexuelle spontanée pendant cette période.

Ces résultats permettent maintenant d'écarter très précocement des animaux porteurs de ce génotype et donc capable de le transmettre à leur descendance, de façon à ne conserver que les animaux possédant une aptitude naturelle au désaisonnement, c'est à dire aptes à une reproduction spontanée à contre-saison, sans mettre en oeuvre des tests sur descendance lourds et coûteux.

La méthode de typage selon l'invention présente plusieurs avantages importants:
- elle ne nécessite qu'une intervention simple, une prise de sang, de la part de l'éleveur,
- la méthode est aujourd'hui facilement mise en routine dans les laboratoires et le résultat du diagnostic peut être obtenu rapidement, en moins de 48 heures,
- enfin et surtout, le résultat permet le testage sur l'ADN génomique dès le sevrage évitant ainsi un entretien coûteux de reproducteurs potentiels dont une partie aurait été éliminée ultérieurement, non sans avoir diffusé les allèles défavorables pour le caractère considéré.

En outre, les résultats donnés ci-dessus apportent un enseignement important à la compréhension des mécanismes moléculaires chez les animaux dont le saisonnement dépend d'un contrôle photopériodique impliquant le récepteur Mel₁ₐ à la mélatonine, comme notamment :
- Le fait que l'une des mutations associée à l'allèle "-", celle en 706, modifie la séquence protéique du récepteur entraînant la possibilité d'une modification fonctionnelle, à la différence de la mutation au niveau du nucléotide en position 612 qui est silencieuse. Il est à noter qu'elle est située dans le 5ème passage transmembranaire du récepteur (isoleucine 220 remplaçant une valine), proche de l'histidine 211 dont la mutation modifie l'affinité de liaison de la mélatonine (Conway et al., 1997).
- Des études de liaison de la mélatonine à des membranes de pars tuberalis issues d'animaux génotypés pour la présence du site MnlI, montrent que cette liaison est plus élevée chez les animaux de génotype "--" que chez les animaux de génotype "++". Cette observation démontre une différence dans les relations hormone-récepteur selon le génotype.

La séquence de l'Exon II du récepteur Mel₁ₐ de référence est celle donnée par Reppert et al. (1994, Neuron, 13, 1177-1185) qui est représentée à la figure 1 en annexe. Il sera fait référence dans ce qui suit à la séquence de la figure 1, qui correspond à la SEQ ID NO 1 donnée dans la liste de séquence en annexe, identique à celle de la figure 1, mais dont la numérotation des nucléotides est décalée de façon a débuter au nucléotide No. 1, et en conséquence dans laquelle plus particulièrement :
- la position No. 453 de la figure 1 correspond à la position No. 171 dans SEQ ID NO.1,
- la position No. 603 de la figure 1 correspond à la position No. 321 dans SEQ ID NO.1,
- la position No. 605 de la figure 1 correspond à la position No. 323 dans SEQ ID NO.1,
- la position No. 609 de la figure 1 correspond à la position No. 327 dans SEQ ID NO.1,
- la position No. 612 de la figure 1 correspond à la position No. 330 dans SEQ ID NO.1.
- la position No. 620 de la figure 1 correspond à la position No. 338 dans SEQ ID NO.1,
- la position No. 706 de la figure 1 correspond à la position No. 424 dans SEQ ID NO.1,
- la position No. 891 de la figure 1 correspond à la position No. 609 dans SEQ ID NO.1.

La présente invention a donc plus particulièrement pour objet une méthode de typage d'un animal photopériodique de préférence pour son aptitude à la reproduction à contre-saison et/ou sa capacité de transmettre cette aptitude à sa descendance, caractérisée en ce que l'on identifie le génotype du site de restriction MnlI polymorphe présent dans la portion de l'Exon II du récepteur Mel₁ₐ situé entre les nucléotides en position 321 et 338 dans la séquence SEQ ID NO.1 chez les ovins, ou dans une séquence homologue chez d'autres espèces.

En effet, cette région apparaît substantiellement conservée dans nombreuses espèces notamment parmi celles citées ci-dessous.

La méthode de l'invention concerne par exemple les animaux choisis parmi les espèces suivantes : ovins, porcins, bovins et caprins. La méthode de l'invention s'applique avantageusement aux animaux dont le saisonnement dépend d'un contrôle photopériodique impliquant le récepteur à la mélatonine, et parmi ceux-ci, plus particulièrement aux ovins et caprins.

La méthode de l'invention appliquée chez les ovins ou les caprins consiste à identifier le génotype du site de restriction MnlI polymorphe situé entre les nucléotides aux positions 330 à 333 dans la séquence SEQ ID NO.1 de l'Exon II du récepteur Mel₁ₐ. La méthode de l'invention consiste alors à détecter la présence d'une mutation au niveau du nucléotide en position 330 dans la séquence SEQ ID NO.1 consistant en le remplacement d'un nucléotide G par un nucléotide A.

Avantageusement, chez les ovins, et chez les espèces dont la portion de l'exon II du récepteur Mel₁ₐ comprenant un site MnlI polymorphe est homologue à celle située entre les nucléotides en position 321 et 338 dans la séquence SEQ ID NO.1, comme les caprins, la méthode de l'invention consiste en outre à identifier des mutations associées au génotype dudit site de restriction MnlI polymorphe, et plus particulièrement au moins une de celles situées au niveau des nucléotides en position 171, 424 et 609 dans la séquence SEQ ID NO.1 définissant avec la mutation située au niveau du nucléotide en position 330 dans la séquence SEQ ID NO.1 un nouvel allèle, désigné précédemment allèle "-".

Ainsi, le génotypage des animaux permet d'éliminer les porteurs du génotype "--" qui ne sont pas aptes à une reproduction à contre-saison, voire "+-" afin d'éliminer l'allèle "-".

La méthode de l'invention peut ainsi consister à identifier sur l'un ou les deux chromosomes parentaux une mutation au niveau du nucléotide en position 330 dans la séquence SEQ ID NO.1 caractérisant la perte du site de restriction MnlI polymorphe présent dans l'exon II du récepteur Mel₁ₐ, et éventuellement au moins une des mutations situées au niveau des nucléotides en position 171, 424 et 609 dans la séquence SEQ ID NO.1. Un animal présentant un génotype "--" aura donc perdu le site MnlI polymorphe de l'Exon II du gène du récepteur Mel₁ₐ sur ces deux chromosomes, et pourra être écarté car il ne présente pas l'aptitude à la reproduction à contre-saison. Un animal présentant un génotype "+-" aura donc perdu le site MnlI polymorphe de l'Exon II du gène du récepteur Mel₁ₐ sur l'un de ces chromosomes, et pourra être éventuellement écarté car il sera susceptible de ne pas transmettre l'aptitude à la reproduction à contre-saison à une partie de sa descendance. Toutefois, on ne peut éliminer d'emblée tous les animaux de génotype "+ -" car ils peuvent représenter 50% de la population. En revanche, à terme, une telle élimination progressive est souhaitable afin d'éviter la reconstitution de génotypes "- -" chez les descendants de parents de génotype "+ -".

Chez les ovins, les inventeurs ont montré que la mutation du nucléotide en position 330 dans la séquence SEQ ID NO.1, modifie le site de reconnaissance de l'enzyme MnlI, et permet donc de sélectionner les animaux susceptibles de présenter une aptitude à la reproduction à contre-saison ou à transmettre cette aptitude à sa descendance. La méthode de l'invention consiste en d'autres termes à mettre en évidence par toute technique appropriée la présence ou l'absence de cette mutation au niveau du nucléotide en position 330 dans la séquence SEQ ID NO.1. Parmi ces méthodes, une technique préférée consiste à analyser le profil de digestion par l'enzyme MnlI d'un échantillon d'ADN obtenu à partir de l'animal tester. La présence ou l'absence d'un ou plusieurs produits de digestion par MnlI indiquent la présence ou l'absence du site correspondant et donc permet de déterminer si l'animal présente une aptitude à la reproduction à contre-saison.

Une forme préférée de réalisation de la méthode de l'invention est caractérisée en ce que :
- on soumet un échantillon d'ADN, obtenu à partir d'un animal, comprenant tout ou partie de l'Exon II du gène du récepteur Mel₁ₐ portant un site de restriction MnlI polymorphe, à l'action de l'enzyme MnlI,
- on détermine le génotype dudit animal à partir de l'analyse des produits de digestion par l'enzyme MnlI.

L'échantillon d'ADN doit comprendre un site de restriction MnlI polymorphe présent dans l'Exon II du gène récepteur Mel₁ₐ à la mélatonine, comme indiqué précédemment, il s'agit de préférence de la portion de l'Exon II du gène du récepteur Mel₁ₐ situé entre les nucléotides en position 321 et 338 de la séquence SEQ ID NO.1 chez les ovins, ou dans une séquence homologue chez d'autres espèces.

L'ADN de l'échantillon est avantageusement de l'ADN génomique, obtenu par exemple à partir des éléments nucléés du sang selon une méthode telle que celle de Miller et al., (Nucleic Acids Research, 1996, 16, 8).

La portion de l'exon II du gène du récepteur Mel₁ₐ portant un site de restriction MnlI polymorphe, est avantageusement amplifiée de préférence par PCR

La mise en évidence de la région d'intérêt peut être réduite à une portion de 600 nucléotides de l'Exon II incluant le site MnlI polymorphe et avantageusement chez les ovins les 4 mutations situées au niveau des nucléotides en position 330, 171, 424 et 609 dans la séquence de la séqence SEQ ID NO.1. En effet, cette portion de l'exon II permet, après génotypage par MnlI, d'effectuer des vérifications éventuelles de la composition nucléotidique par séquençage.

Les produits d'amplification sont digérés par l'enzyme MnlI, puis les produits de digestion sont dénaturés puis séparés par exemple par électrophorèse en gel d'agarose, et le génotype de l'animal est déterminé à partir de l'analyse de la taille des produits de digestion.

La digestion du produit d'amplification, environ 1 µg, par MnlI est complète avec 1-2 unités d'enzyme pendant 2 ou 3 heures, voire une nuit pour commodité. Les produits de digestion sont ensuite dénaturés puis séparés par électrophorèse en gel d'agarose.

Le génotype de l'animal est déterminé à partir de l'analyse des produits de digestion par l'enzyme MnlI. Les produits de digestion peuvent être mis en évidence par plusieurs méthodes dont l'une facile à mettre en oeuvre utilise la fixation du bromure d'éthidium révélé sous UV. Une photographie est prise comme document de fichier. Sous UV comme sur la photographie, la taille des produits de digestion est évaluée par comparaison à une échelle de marqueurs.

Dans le cas des ovins et conformément aux exemples détaillés dans la partie expérimentale ci-après, la présence du site MnlI correspond à une bande de 236 nucléotides située juste au-dessus d'une bande de 216 nucléotides non polymorphique; elle est associée à une bande de 67 nucléotides souvent non visible dans les conditions expérimentales usuelles. La perte du site correspond à la somme des deux bandes précédentes soit 303 nucléotides, bien visible car seule de cette taille. L'analyse des produits de digestion selon la méthode de l'invention est donc basée sur l'identification des trois types de résultats possibles observés par les Inventeurs : (i) la présence d'une bande à 236 nucléotides (et rien en 303) correspond au génotype "++", (ii) la présence d'une bande de 303 nucléotides (et rien en 236) correspond au génotype "--", (iii) la présence d'une bande de 236 nucléotides et une autre de 303 nucléotides révèlent un génotype "+ -".

D'autres avantages et caractéristiques de l'invention apparaîtront des exemples qui suivent décrivant le génotypage du récepteur Mel₁ₐ chez le mouton et la mise en oeuvre de la méthode de l'invention pour sélectionner les animaux destinés à la reproduction.

### Exemple I : Génotypage et activité sexuelle spontanée en contre-saison.

### 1) Essai 1.

Animaux étudiés : Moutons. 933 brebis Mérinos d'Arles sont testées pour la présence ou non d'une activité ovulatoire cyclique spontanée au printemps. Durant la première quinzaine d'avril, deux échantillons de sang sont collectés à 8-10 jours d'intervalle et la progestérone plasmatique est mesurée par radio-immunologie. La présence de taux de progestérone supérieurs à 1 ng/ml dans l'un des deux échantillons révèle la présence d'un corps jaune ovarien et donc la présence de cyclicité. Cette procédure a été effectuée trois années consécutives entre avril 1995 et avril 1997 sur la majorité des brebis. Un total de 71 animaux a été sélectionné pour le génotypage, 35 sans activité sexuelle durant la période expérimentale et 36 présentant une telle activité.

Le génotypage révèle une distribution des fréquences des génotypes très significativement différente selon que les animaux sont en anoestrus ou spontanément cycliques (P<0.001). En particulier, le génotype "--" n'a été observé chez aucun animal cyclique alors qu'il représente 28,5 % de l'effectif des animaux non cycliques (P<0,001).

### 2) Essai 2.

Animaux étudiés: Moutons. Il a été testé la fréquence du génotype "--" chez une race considérée comme beaucoup plus saisonnée que la race Mérinos d'Arles, la race Ile-de-France (Thimonier et Mauléon, 1969, Ann. Biol. anim., Biochim., Biophys., 9, 233-250). Vingt-neuf brebis ont été sélectionnées parmi les filles de béliers préalablement génotypés "+ -" afin d'éviter un biais qui pourrait être dû à des conditions particulières du troupeau. L'hypothèse attendue d'une plus grande fréquence de l'allèle "-" dans cette race plus saisonnée s'est trouvée vérifiée avec la présence de 38% de brebis de génotype "--".

### 3) Vérifications.

Des séquençages (n = 48) de l'exon II entre les nucléotides aux positions 2 à 827 de la séquence ID NO.1, soit la quasi totalité de l'exon II, après clonage ou directement après PCR sur une portion plus réduite, entre les nucléotides aux positions 70 à 665 de la séquence ID NO.1, chez des génotypes homozygotes, ont vérifié l'exactitude des données obtenues par génotypage avec l'enzyme MnlI.

En outre, ces séquençages ont permis d'établir que dans les limites de la séquence étudiée, l'allèle "-" représentait un jeu unique des 4 mêmes mutations (n= 18) .

### Exemple II : Détermination du génotype.

### 1) Préparation de l'ADN génomique.

L'ADN génomique est préparé à partir d'un échantillon de sang de 10 ml collecté sur EDTA à partir de la veine jugulaire et congelé pour rompre les globules rouges anucléés. Après décongélation de l'échantillon, celui-ci est transféré dans un tube de 40-50 ml et complété par du tampon (Tris HCl 10 mM pH 7.6, MgCl₂ 5 mM, NaCl 10 mM). Après une première centrifugation à 1200 t/mn pendant 10 mn, la phase aqueuse est décantée et les globules blancs remis en solution dans le tampon précédent. La nouvelle centrifugation est de 1000 t/mn pendant 7 mn. Un nouveau lavage identique est réalisé.

Après décantation, les globules blancs sont récupérés par deux fois 1,25 ml de tampon Tris HCl 10 mM pH 7.6, EDTA 0,1%, auxquels sont additionnés 100 µl de protéinase K (10 mg/ml), 250 µl d'EDTA 0,5 M pH 8 et 250 µl SDS 10%.

### Agitation douce 2 h à 50°C.

Addition de 2,15 ml NaCl 6M et centrifugation à 3800 t/mn pendant 20 mn. La phase liquide, qui contient les acides nucléiques, est additionnée de 2,5 vol d'éthanol absolu et l'ADN précipite en forme de filaments. Après 3 lavages par l'alcool à 70°, le précipité est brièvement séché à l'air et transféré dans 1 ml de tampon Tris/EDTA indiqué ci-dessus. La densité optique à 260 nM est lue sur 20 µl d'échantillon après extraction d'une nuit. La concentration est usuellement comprise entre 50 et 150 ng/µl. Cet ADN se conserve à 4°C pendant plusieurs années.

### 2) Réaction de polymérisation en chaîne (PCR).

Les primers utilisés sont les suivants :
Primer sens 5' < GCGTCTATAGTTAACAATGG > 3'
Primer antisens 5' <GCCATATAGTAACTAGCCAC > 3'

Les conditions de la PCR sont les suivantes en utilisant des réactifs standards de bonne qualité (Boehringer, Pharmacia, Biolabs, Promega) :
- ADN génomique quantité 100-150 ng, # 1-2 µl,
- 2 µl d'une solution de dNTP (dATP, dCTP, dGTP, dTTP, chacun à 5 mM),
- 1 µl primer sens et 1 µl primer antisens (chacun à 10 pmole/µl),
- 1,5 µl de tampon Tris 50 mM, KCl 75 mM, MgCl2 3 mM,
- 1,5 µl MgCl2 25 mM (concentration finale 3 mM),
- 0,5 µl DTT 0,1 M,
- 5 µl de Tampon pour PCR 10 X (fourni avec l'enzyme),
- 0,2 µl d'enzyme Taq Polymérase (= 1 U),
- H2O : qsp 50 µl,

Le tampon de l'enzyme et l'enzyme elle-même sont mélangés ensemble et ce mix est additionné aux autres réactifs préalablement pipettés dans un tube pour PCR.

Le tout est recouvert de 80 µl d'huile minérale.

La PCR elle-même consiste en 35 cycles selon le programme suivant :
Cycle 1 : dénaturation 3 mn à 95°C -- couplage 1 mn à 62°C -- élongation 2 mn à 72°C,
Cycles 2-35 : dénaturation 1 mn à 95°C -- couplage 1 mn à 62°C -- élongation 2 mn à 72°C
- La PCR se termine par une élongation finale de 10 mn à 72°C.

### 3) Digestion enzymatique.

Les réactifs sont additionnés dans l'ordre : 39 µl H2O + 32 µl produit de PCR + 8 µl de tampon fourni avec l'enzyme + 0,8 µl BSA (10 mg/ml) + 0,2 µl Enzyme MnlI (= 1U). La digestion est commodément effectuée une nuit à 37°C. L'échantillon est ensuite concentré dans un bain-marie sec à 65°C pour être réduit à 25 µl environ (une concentration par speedvac est également possible).

### 4) Identification des produits de digestion en gel d'agarose.

Un gel d'agarose à 3% (NuSieve, FMC Bioproducts) est préparé dans du tampon 45 mM tris-borate, 1 mM EDTA (tampon 0,5 X). Les échantillons sont additionnés de 1 µl de solution de bleu de bromophénol (2,5 mg de bleu de bromophénol, saccharose 40%, EDTA 10 mM pH 8, NaOH 20 mM qsp 1 ml) et chauffés à 65°C pendant 5-7 mn ainsi qu'un échantillon de 5 µl de marqueur de poids moléculaire de DNA étalonné par 100 paires de base jusqu'à 1 kb (InVitrogene). Les échantillons sont soumis à une électrophorèse de 100 V et après environ 6 cm de migration du bleu de bromophénol, le gel d'agarose est coloré par le bromure d'éthidium (10 µg/ml) pendant 5-10 mn.

Le gel est photographié sous UV, donnant la présence des bandes de digestion.

La méthode de l'invention conduit à éliminer les animaux dont l'ADN génomique ne présente que la seule bande de 303 bp du lot des reproducteurs (génotype "--"). Dans un second temps, il peut être procédé à une élimination des animaux porteurs du génotype "+ -" (bandes de 303 et 236 bp simultanément présentes).

## Revendications

1. Méthode de typage d'un animal photopériodique pour une fonction physiologique saisonnière et/ou sa capacité à transmettre cette fonction physiologique saisonnière à sa descendance, **caractérisée en ce que** l'on identifie le génotype d'un site de restriction MnlI polymorphe présent dans l'Exon II du récepteur Mel₁ₐ à 1a mélatonine, une mutation au niveau du site de reconnaissance de ladite enzyme permettant de sélectionner les animaux susceptibles de présenter la fonction physiologique saisonnière et/ou de transmettre cette aptitude à sa descendance.

2. Méthode de typage d'un animal photopériodique pour une fonction physiologique saisonnière et/ou sa capacité à transmettre cette fonction physiologique saisonnière à sa descendance selon la revendication 1, **caractérisée en ce que** ladite fonction physiologique saisonnière est l'aptitude à 1a reproduction à contre-saison.

3. Méthode de typage selon l'une des revendications 1 ou 2, **caractérisée en ce que** l'on identifie le génotype du site de restriction MnlI polymorphe présent dans la portion de l'Exon II du récepteur Mel₁ₒ situé entre les nucléotides en position 321 et 338 dans la séquence SEQ ID NO.1 chez les ovins, ou dans une séquence homologue chez d'autres espèces.

4. Méthode de typage selon l'une des revendications 1 à 3, **caractérisée en ce que** l'animal appartient à l'une des espèces suivantes ovins, caprins, porcins et bovins.

5. Méthode de typage selon l'une quelconque des revendications 1 à 4, **caractérisée en ce que** l'animal est choisi parmi les ovins et les caprins, et **en ce que** l'on identifie le génotype du site de restriction MnlI polymorphe situés entre les nucléotides aux positions 330 à 333 dans la séquence SEQ ID NO.1 de l'Exon II du récepteur Mel₁ₐ.

6. Méthode de typage selon la revendication 5, **caractérisée en ce que** l'on identifie la présence d'une mutation au niveau du nucléotide en position 330 dans la séquence SEQ ID NO.1 consistant en le remplacement d'un nucléotide G par un nucléotide A.

7. Méthode de typage selon l'une des revendications 5 ou 6, **caractérisée en ce que** l'on identifie en outre au moins un mutation située au niveau des nucléotides en position 171, 424 et 609 dans la séquence SEQ ID NO.1 définissant avec la mutation située au niveau du nucléotide en position 330, l'allèle "-".

8. Méthode de typage selon l'une des revendications 5 à 7, **caractérisée en ce que** identifie 1a présence d'une desdites mutations sur l'un ou les deux chromosomes parentaux.

9. Méthode de typage selon l'une quelconque des revendications 1 à 8, **caractérisée en ce que** :
- on soumet un échantillon d'ADN, obtenu à partir dudit animal, comprenant tout ou partie de l'Exon II du gène du récepteur Mel₁ₙ portant un site de restriction MnlI polymorphe, à l'action de l'enzyme MnlI,
- on détermine le génotype dudit animal à partir de l'analyse des produits de digestion par l'enzyme MnlI.

10. Méthode de typage selon la revendication 9, **caractérisée en ce que** l'échantillon d'ADN comprend la portion de l'Exon II du gène du récepteur Mel₁ₐ situé entre les nucléotides en position 321 et 338 de la séquence SEQ ID NO.1 chez les ovins, ou dans une séquence homologue chez d'autres espèces, avantageusement amplifiée par PCR.

11. Méthode de typage selon l'une quelconque des revendications 9 à 10, **caractérisée en ce que** l'échantillon d'ADN comprend une portion de 600 nucléotides de l'Exon II incluant le site MnlI polymorphe, avantageusement amplifiée par PCR.

12. Méthode de typage selon l'une quelconque des revendications 9 à 10, **caractérisée en ce que** l'ADN de l'échantillon avantageusement amplifié est digéré par l'enzyme MnlI, puis les produits de digestion sont dénaturés puis séparés par exemple par électrophorèse en gel d'agarose, et le génotype de l'animal est déterminé à partir de l'analyse de la taille des produits de digestion.

13. Méthode de typage selon la revendication 12, **caractérisée en ce que** l'animal est un ovin et **en ce que** l'analyse consiste à identifier l'un des trois profils de digestion suivants:
(i) la présence d'une bande à 236 nucléotides (et rien en 303) correspond au génotype "++",
(ii) la présence d'une bande de 303 nucléotides (et rien en 236) correspond au génotype "--",
(iii) la présence d'une bande de 236 nucléotides et une autre de 303 nucléotides révèlent un génotype "+ -",
à partir de l'ADN de l'échantillon amplifié par PCR en utilisant le primer sens 5' < GCGTCTATAGTTAACAATGG > 3' et le primer antisens 5' <GCCATATAGTAACTAGCCAC > 3'.

## Patentansprüche

1. Methode zur Typisierung eines fotoperiodischen Tiers für eine saisonale physiologische Funktion und/oder seine Fähigkeit zur Weitergabe dieser saisonalen physiologischen Funktion an seine Nachkommen, **dadurch gekennzeichnet, dass** man den Genotyp einer polymorphen Restriktionsstelle MnlI in Exon II des Melatoninrezeptors Mel₁ₐ identifiziert, wobei anhand einer Mutation auf der Erkennungsstelle des besagten Enzyms die Tiere selektiert werden können, die möglicherweise die saisonale physiologische Funktion aufweisen und/oder diese Fähigkeit an ihre Nachkommen weitergeben.

2. Methode zur Typisierung eines fotoperiodischen Tiers für eine saisonale physiologische Funktion und/oder seine Fähigkeit zur Weitergabe dieser saisonalen physiologischen Funktion an seine Nachkommen nach Anspruch 1, **dadurch gekennzeichnet, dass** die besagte saisonale physiologische Funktion die Fähigkeit zur asaisonalen Reproduktion ist.

3. Methode zur Typisierung nach einem der Ansprüche 1 oder 2, **dadurch gekennzeichnet, dass** man den Genotyp der polymorphen Restriktionsstelle MnlI im Abschnitt von Exon II des Rezeptors Mel₁ₐ zwischen den Nukleotidpositionen 321 und 338 in der Sequenz SEQ ID NO.1 bei Schafen oder in einer entsprechenden Sequenz bei anderen Arten identifiziert.

4. Methode zur Typisierung nach einem der Ansprüche 1 bis 3, **dadurch gekennzeichnet, dass** das Tier zu einer der folgenden Arten Schaf, Ziege, Schwein und Rind gehört.

5. Methode zur Typisierung nach einem der Ansprüche 1 bis 4, **dadurch gekennzeichnet, dass** das Tier ein Schaf oder eine Ziege ist, und dadurch, dass man den Genotyp der polymorphen Restriktionsstelle MnlI zwischen den Nukleotidpositionen 330 bis 333 in der Sequenz SEQ ID NR.1 von Exon II des Rezeptors Mel₁ₐ identifiziert.

6. Methode zur Typisierung nach Anspruch 5, **dadurch gekennzeichnet, dass** man das Vorhandensein einer Mutation an der Nukleotidposition 330 in der Sequenz SEQ ID NO.1 identifiziert, bestehend aus der Ersetzung eines G-Nukleotids durch einen A-Nukleotid.

7. Methode zur Typisierung nach einem der Ansprüche 5 oder 6, **dadurch gekennzeichnet, dass** man außerdem mindestens eine Mutation an den Nukleotidpositionen 171, 424 und 609 in der Sequenz SEQ ID NO.1 identifiziert, die mit der Mutation an der Nukleotidposition 330 das Allel "_" definiert.

8. Methode zur Typisierung nach einem der Ansprüche 5 bis 7, **dadurch gekennzeichnet, dass** man das Vorhandensein einer der besagten Mutationen auf einem Chromosom oder beiden elterlichen Chromosomen identifiziert.

9. Methode zur Typisierung nach einem der Ansprüche 1 bis 8, **gekennzeichnet dadurch, dass**:
- man eine DNA-Probe, die besagtem Tier entnommen wurde und die Exon II des Gens des Rezeptors Mel₁ₐ mit einer polymorphen Restriktionsstelle MnlI ganz oder teilweise aufweist, der Wirkung des Enzyms MnlI aussetzt,
- man den Genotyp des besagten Tiers durch Analyse der Verdauprodukte des Enzyms MnlI festgestellt.

10. Methode zur Typisierung nach Anspruch 9, **dadurch gekennzeichnet, dass** die DNA-Probe den Abschnitt von Exon II des Gens des Rezeptors Mel₁ₐ zwischen den Nukleotidpositionen 321 und 338 in der Sequenz SEQ ID NR.1 bei Schafen aufweist, oder in einer entsprechenden Sequenz bei anderen Arten, vorzugsweise verstärkt durch PCR.

11. Methode zur Typisierung nach einem der Ansprüche 9 bis 10, **dadurch gekennzeichnet, dass** die DNA-Probe einen Abschnitt von 600 Nukleotiden von Exon II aufweist, der die polymorphe Stelle MnlI einschließt, vorzugsweise verstärkt durch PCR.

12. Methode zur Typisierung nach einem der Ansprüche 9 bis 10, **dadurch gekennzeichnet, dass** die vorzugsweise verstärkte DNA-Probe vom Enzym MnlI verdaut wird, die Verdauprodukte danach zum Beispiel durch Elektrophorese in Agarose-Gel denaturiert und separiert werden und der Genotyp des Tiers anhand der Analyse der Größe der Verdauprodukte bestimmt wird.

13. Methode zur Typisierung nach Anspruch 12, **dadurch gekennzeichnet, dass** das Tier ein Schaf ist und dadurch, dass die Analyse in der Identifizierung eines der drei folgenden Verdauungsprofile besteht:
(i) das Vorhandensein einer Bande mit 236 Nukleotiden (und keiner mit 303) entspricht dem Genotyp "++",
(ii) das Vorhandensein einer Bande mit 303 Nukleotiden (und keiner mit 236) entspricht dem Genotyp "-",
(iii) das Vorhandensein einer Bande mit 236 Nukleotiden und einer anderen mit 303 Nukleotiden offenbart einen Genotyp "+-",
ausgehend von einer PCR-verstärkten DNA-Probe bei Verwendung des Sense-Primers 5' <GCGTCTATAGTTAACAATGG> 3' und des Antisense-Primers 5' <GCCATATAGTAACTAGCCAC> 3'.

## Claims

1. Method for typing a photoperiodic animal for a seasonal physiological function and/or its capacity to transmit this seasonal physiological function to its offspring, **characterised in that** the genotype of a polymorphic MnlI restriction site present in the Exon II of the melatonin receptor Mel₁ₐ is identified, a mutation within the recognition site of said enzyme allowing the selection of animals likely to display the seasonal physiological function and/or to transmit this aptitude to its offspring.

2. Method for typing a photoperiodic animal for a seasonal physiological function and/or its capacity to transmit this seasonal physiological function to its offspring, according to claim 1, **characterised in that** said seasonal physiological function is the aptitude to reproduce out of season.

3. Method for typing according to one of claims 1 or 2, **characterised in that** the genotype of the polymorphic MnlI restriction site present in the portion of the Exon II of the receptor Mel₁ₐ situated between the nucleotides in positions 321 and 338 in the SEQ ID NO.1 sequence in ovines, or in a homologous sequence in other species, is identified.

4. Method for typing according to one of claims 1 to 3, **characterised in that** the animal belongs to one of the following species: ovines, caprines, porcines and bovines.

5. Method for typing according to any one of claims 1 to 4, **characterised in that** the animal is chosen from among the ovines and the caprines, and **in that** the genotype of the polymorphic MnlI restriction site situated between the nucleotides at positions 330 to 333 in the SEQ ID NO.1 sequence of the Exon II of the receptor Mel₁ₐ, is identified.

6. Method for typing according to claim 5, **characterised in that** the presence of a mutation affecting the nucleotide in position 330 in the SEQ ID NO.1 sequence consisting of the replacement of a G nucleotide with an A nucleotide, is identified.

7. Method for typing according to one of claims 5 or 6,
**characterised in that** at least one mutation affecting the nucleotides in positions 171, 424 and 609 in the SEQ ID NO.1 defining, with the mutation affecting the nucleotide in position 330, the allele "-", is further identified.

8. Method for typing according to one of claims 5 to 7, **characterised in that** the presence of one of said mutations on one or both parental chromosomes is identified.

9. Method for typing according to any one of claims 1 to 8, **characterised in that**:
- a DNA sample obtained from said animal, comprising all or part of the Exon II of the melatonin receptor Mel₁ₐ gene carrying a MnlI polymorphic restriction site, is subjected to the action of the enzyme MnlI,
- the genotype of the said animal is determined by analysis of the products of digestion by the enzyme MnlI.

10. Method for typing according to claim 9, **characterised in that** the DNA sample comprises the portion of the Exon II of the receptor Mel₁ₐ gene situated between the nucleotides in positions 321 and 338 of the SEQ ID NO.1 sequence in ovines, or in a homologous sequence in other species, advantageously amplified by PCR.

11. Method for typing according to any one of claims 9 to 10, **characterised in that** the DNA sample comprises a segment of 600 nucleotides from the Exon II including the polymorphic MnlI site, advantageously amplified by PCR.

12. Method for typing according to any one of claims 9 to 10, **characterised in that** the sample DNA, advantageously amplified by PCR, is digested by the enzyme MnlI, then the digestion products are denatured then separated by, for example, agarose gel electrophoresis, and the genotype of the animal is determined from an analysis of the size of the digestion products.

13. Method for typing according to claim 12, **characterised in that** the animal is an ovine and **in that** the analysis consists of identifying one of the following three digestion profiles:
(i) the presence of a band at 236 nucleotides (and nothing at 303) corresponds to the genotype "++",
(ii) the presence of a band of 303 nucleotides (and nothing at 236) corresponds to the genotype "--",
(iii) the presence of a band of 236 nucleotides and another of 303 nucleotides reveals a genotype "+-",
based on the DNA of the sample amplified by PCR using the sense primer 5'<GCGTCTATAGTTAACAATGG>3' and the antisense primer 5'<GCCATATAGTAACTAGCCAC>3'.


### LISTE DE SEQUENCES

<110> Institut national de la recherche Agronomique - IN

<120> Méthode de typage d'un animal photopériodique pour une fonction physiologique saisonnière comme son aptitude à la réproduction à contre-saison.

<130> 5160pct-juin2000

<140> wo2000-xxxxx
<141> 2000-06-28

<150> FR99/08238
<151> 1999-06-28

<160> 2

<170> PatentIn Ver. 2.1

<210> 1
<211> 867
<212> ADN
<213> ovins bovins caprins

<220>
<221> misc_feature
<222> (1)..(867)
<223> EXON II du récepteur Mella de la melatonine 
<400> 1
aatgtgtttg tggtgagcct ggcagttgca gacctgctgg tggccgtgta tccgtacccc 60
ttggcgctgg cgtctatagt taacaatggg tggagcctga gctccctgca ttgccaactt 120
agtggcttcc tgatgggctt gagcgtcatc gggtccgttt tcagcatcac tggaattgcc 180
atcaaccgct attgctgcat ctgccacagc ctcagatacg gcaagctgta tagcggcacg 240
aattccctct gctacgtgtt cctgatctgg acgctgacgc tcgtggcgat cgtgcccaac 300
ctgtgtgtgg ggaccctgca gtacgaccca aggatctatt cctgtacctt cacgcagtcc 360
gtcagctcag cctacacgat cgccgtggtg gtgttccatt tcatagttcc gatgctcgta 420
gtcatcttct gttacctgag aatctgggcc ctggttcttc aggtcagatg gaaggtgaaa 480
ccggacaaca aaccgaaact gaagccccag gacttcagga attttgtcac catgtttgtg 540
gtttttgtcc tctttgccat ttgctgggct cctctgaact tcattggtct cgttgtggcc 600
tcggaccctg ccagcatggc acccaggatc cccgagtggc tgtttgtggc tagttactat 660
atggcatatt tcaacagctg cctcaatgcg atcatatatg gactactgaa ccaaaatttc 720
aggcaggaat acagaaaaat tatagtctca ttgtgtacca ccaagatgtt ctttgtggat 780
agctccaatc atgtagcaga tagaattaaa cgcaaaccct ctccattaat agccaaccat 840
aacctaataa aggtggactc cgtttaa 867
<210> 2
<211> 867
<212> ADN
<213> ovins

<220>
<223> La position N°453 de la figure 1 correspond à la position No. 171 dans SEQ ID No.1

<220>
<223> La position No.603 de la figure 1 correspond à la position No.321 dans SEQ ID No.1

<220>
<223> La position No.605 de la figure 1 correspond à la position No. 323 dans SEQ ID No. 1

<220>
<223> La position No.609 de la figure 1 correspond à 1a position No. 327 dans SEQ ID No. 1

<220>
<223> La position No.612 de la figure 1 correspond à la position No. 330 dans SEQ ID No. 1

<220>
<223> La position No.620 de la figure 1 correspond à la position No. 338 dans SEQ ID No. 1

<220>
<223> La position No.706 de la figure 1 correspond à la position No. 424 dans SEQ ID No. 1

<220>
<223> La position No. 891 de la figure 1 correspond à la position N° 609 dans SEQ ID No. 1

<220>
<221> misc_feature
<222> (1)..(867)
<223> Séquence correspondante à celle comprise entre les bases 283 et 1149 de l'Exon II du récepteur Mel la de la melatonine chez les ovins et représentée en figure 1

<300>
<303> Neuron
<304> 13

<306> 1177-1185
<307> Novembre 1994

<400> 2
aatgtgtttg tggtgagcct ggcagttgca gacctgctgg tggccgtgta tccgtacccc 60
ttggcgctgg cgtctatagt taacaatggg tggagcctga gctccctgca ttgccaactt 120
agtggcttcc tgatgggctt gagcgtcatc gggtccgttt tcagcatcac tggaattgcc 180
atcaaccgct attgctgcat ctgccacagc ctcagatacg gcaagctgta tagcggcacg 240
aattccctct gctacgtgtt cctgatctgg acgctgacgc tcgtggcgat cgtgcccaac 300
ctgtgtgtgg ggaccctgca gtacgaccca aggatctatt cctgtacctt cacgcagtcc 360
gtcagctcag cctacacgat cgccgtggtg gtgttccatt tcatagttcc gatgctcgta 420
gtcatcttct gttacctgag aatctgggcc ctggttcttc aggtcagatg gaaggtgaaa 480
ccggacaaca aaccgaaact gaagccccag gacttcagga attttgtcac catgtttgtg 540
gtttttgtcc tctttgccat ttgctgggct cctctgaact tcattggtct cgttgtggcc 600
tcggaccctg ccagcatggc acccaggatc cccgagtggc tgtttgtggc tagttactat 660
atggcatatt tcaacagctg cctcaatgcg atcatatatg gactactgaa ccaaaatttc 720
aggcaggaat acagaaaaat tatagtctca ttgtgtacca ccaagatgtt ctttgtggat 780
agctccaatc atgtagcaga tagaattaaa cgcaaaccct ctccattaat agccaaccat 840
aacctaataa aggtggactc cgtttaa 867